# EUROPEAN PATENT APPLICATION

(11) **EP 3 311 874 A1**
(43) Date of publication of application: **25.04.2018**
(21) Application number: 17196897.7
(22) Date of filing: 17.10.2017
(51) Int. Cl.: A61M 25/10

(54) **SHAPE CONTROLLED BALLOON CATHETER**

(30) Priority: 17.10.2016 US 201662408855 P
(71) Applicant: Cook Medical Technologies LLC, Bloomington, IN 47404 (US)
(72) Inventor: MAYLE, Brent, Spencer, IN 47460 (US); MERK, James, Terre Haute, IN 47802 (US); SPINDLER, Ralf, Bloomington, IN 47401 (US)
(74) Representative: Garratt, Peter Douglas

(57) **Abstract**

A shape controlled balloon catheter 10 includes the balloon 11 mounted about a catheter 12. A wire 20 is spirally wound about the balloon and includes a manipulation segment 21 extending along a centerline 22 of the catheter away from the balloon. A position of the wire on an outer surface of the balloon changes responsive to movement of the manipulation segment relative to the catheter along the centerline. The balloon has a continuum of different inflated shapes corresponding to a continuum of different positions of the manipulation segment of the wire relative to the catheter.

## Description

### Technical Field

The present disclosure relates generally to balloon catheters, and more particularly to changing the shape of a balloon catheter by manipulating a wire spirally wrapped around the balloon.

### Background

Current balloon catheters often lack a certain amount of conformability when being expanded in curved vasculature. For instance, inflation of typical balloon catheters often tends to straighten a vessel against its natural curvature instead of the balloon conforming to the natural curvature of the vessel. U.S. Patent Publication 2014/0135891 teaches a balloon catheter for curved vessels that includes a pair of manipulation wires attached at various locations to a helical wire wrapped about a balloon. Although this reference recognizes a need for balloon catheters to conform to curved vasculature, the complex three wire structure appears unsuitable for mass production with consistent results.

The present disclosure is directed toward one or more of the problems set forth above.

### Summary

In one aspect, a shape controlled balloon catheter includes a balloon mounted about a catheter. A wire is spirally wound about the balloon and includes a manipulation segment extending along a centerline of the catheter away from the balloon. A position of the wire on an outer surface of the balloon changes responsive to movement of the manipulation segment relative to the catheter along the centerline. The balloon has a continuum of different inflated shapes corresponding to a continuum of different positions of the manipulation segment relative to the catheter along the centerline.

In another aspect, a method of operating a shape controlled balloon catheter includes positioning a balloon of the shaped controlled balloon catheter at a site in a passageway while in a deflated state. The balloon is inflated. The balloon is constrained during inflation with a wire spirally wound about the balloon so that the inflated balloon has a first shape. The inflated balloon is changed from the first shape to a second shape responsive to movement of a manipulation segment of the wire along a centerline of the catheter away from the balloon.

### Brief Description of the Drawings

Fig. 1 is a side schematic view of a shape controlled balloon catheter in a deflated state;
Fig. 2 is a side schematic view of the balloon catheter of Fig. 1 in a deployed uninflated state;
Fig. 3 is a side schematic view of the balloon catheter of Figs. 1 and 2 inflated to a first shape;
Fig. 4 is a side schematic view of the balloon catheter of Fig. 3 after a spirally wound wire has been moved to change the inflated balloon to a second shape;
Fig. 5 is a side schematic view of the balloon catheter of Figs. 3 and 4 after further movement of the wire to still another inflated shape along a continuum of different inflated shapes;
Fig. 6 is a side schematic view of the balloon catheter of Fig. 5 in an unconstrained inflated shape;
Fig. 7 is a side schematic view of the balloon catheter of Figs. 1-6 deflated and withdrawn into a sheath;
Fig. 8 is a side schematic view of a shape controlled balloon catheter according to another embodiment of the present disclosure;
Fig. 9 is a sectioned view of the balloon catheter as viewed along section lines 9-9 of Fig. 8;
Fig. 10 shows a distal segment of a wire with a pre-set spiral shape;
Fig. 11 is a side view of the wire of Fig. 10 after the shape has changed responsive to a temperature increase;
Fig. 12 shows a shape controlled balloon catheter according to the present disclosure being positioned at a site in a curved passageway in a deflated state;
Fig. 13 is a schematic view of the balloon catheter of Fig. 12 inflated to a first shape;
Fig. 14 shows the balloon catheter of Fig. 13 after the spirally wound wire has been moved to cause the balloon to assume a second inflated shape; and
Fig. 15 shows the balloon catheter of Fig. 14 after the spiral wire has been withdrawn away from the balloon.

### Detailed Description

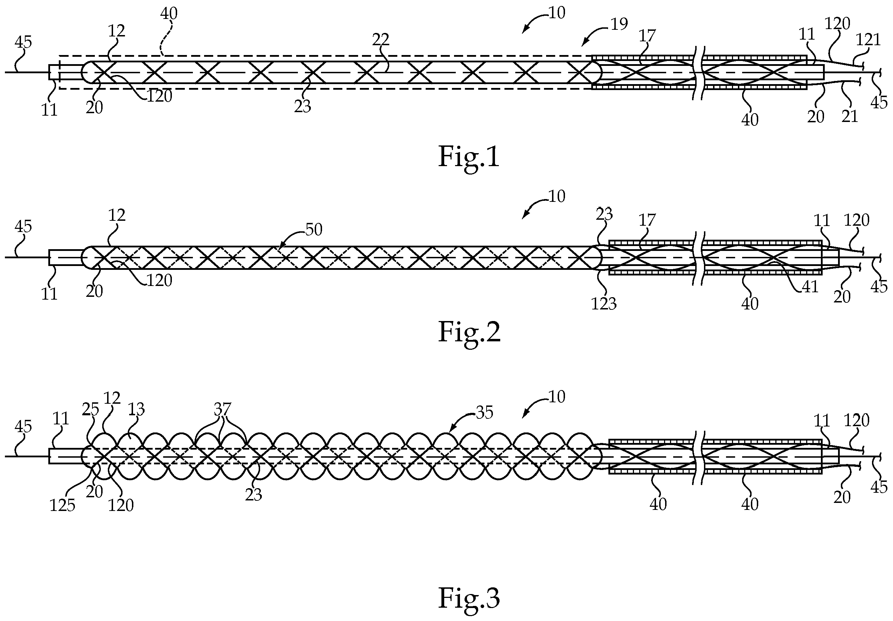

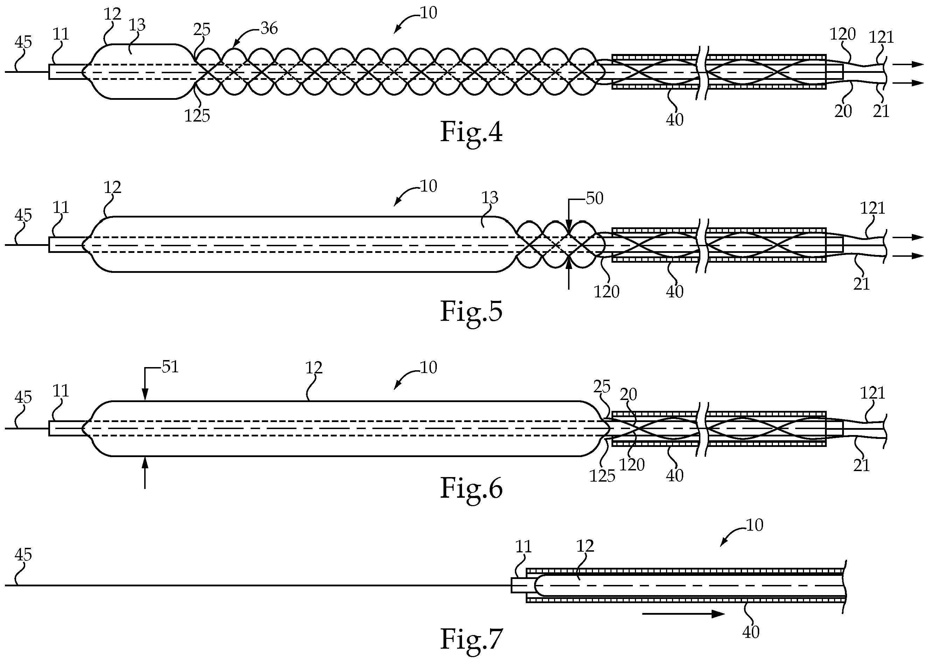

Referring initially to Figs. 1-7, a shape controlled balloon catheter 10 includes a catheter 11 with a balloon 12 mounted about the catheter. As best shown in Fig. 6, if unconstrained, balloon 12 would assume a typical regular cylindrical shape upon inflation. However, shape control balloon catheter 10 includes a wire 20 spirally wound about the balloon 12. Except for wire 20, balloon catheter 10 could have a typical structure well known in the art. The wire 20 includes a manipulation segment 21 extending along a centerline 22 of the catheter 11 away from the balloon 12. Preferably the manipulation segment 21 would be available for manipulation by a clinician at an end of the balloon catheter 10 remote from balloon 12. In the illustrated embodiment, shape controlled balloon catheter 10 also includes a second wire 120 spirally wound about the balloon. Although second wire 120 is wound in an opposite direction about the balloon 12 than the first wire 20, those skilled in the art will appreciate that the two wires could be wound in the same direction to create a double helix. Like the first wire 20, second wire 120 includes a manipulation segment 121 extending along centerline 22 away from balloon 12. A position of the wire 20, 120 on an outer surface 13 of the balloon 12 changes responsive to a movement of the manipulation segment 21, 121 relative to the catheter 11 along the centerline 22. In this embodiment, the distal end 25, 125 is unattached to either balloon 12 or catheter 11 so that the distal end 25, 125 of the wire is moved along the outer surface 13 of the balloon 12, both around balloon 12 and along centerline 22, responsive to movement of the manipulation segment 21, 121 relative to the catheter 11 along centerline 22. The manipulation segments 21, 121 can be moved individually or simultaneously. The balloon 12 has a continuum of different inflated shapes corresponding to a continuum of different positions of the manipulation segment 21, 121 relative to the catheter 11 along centerline 22. Figs. 3, 4, 5 and 6 show four different of the continuum of different inflated shapes corresponding to different positioning of the manipulation segment 21, 121 away from balloon 12.

Although shape controlled balloon catheter 10 could be maneuvered to a treatment site alone over a wire guide 45, balloon catheter 10 may be maneuvered to a treatment site simultaneously with a sheath 40. As shown in Fig. 1, the sheath 40 may not cover balloon 12 when the balloon catheter 10 is in its deflated state 19 or the sheath may cover balloon 12, as shown with the dashed line 40. After arriving at a treatment site, as shown in Fig. 2, the sheath, if included, may be withdrawn out of contact with balloon 12 in preparation for its inflation. In this embodiment, the manipulation segment 21, 121 of the wires 20, 120, extends along the centerline 22 between an outer surface 17 of the catheter 11 and an inner surface 41 of the sheath 40. Catheter 11 would be slidably received in sheath 40, if included.

In order to hold the balloon 12 in the shape shown in Fig. 3 against the inflation pressure within balloon 12, the wires 20, 120 may have distal segments 23 that have a pre-set spiral shape 50 with a diameter that is smaller than an inflation diameter 51 of the balloon 12. When the balloon 12 is inflated to a first shape 35 as shown in Fig. 3, the balloon 12 becomes highly conformable to curved passageways by having the ability to bend about creases 37 that are defined by the respective wires 20, 120. After conforming to the curved passageway, the wires 20, 120 may be pulled away from balloon 12 after the balloon catheter 10 has already conformed to a curved passageway to then fully inflate the balloon as shown in Fig. 6. After a procedure is performed, the balloon 12 may be deflated and withdrawn into sheath 40 and moved away from a treatment site as shown in Fig. 7. Although the shape controlled balloon catheter 10 of Figs. 1-7 is shown without a balloon expanded stent, those skilled in the art will appreciate that balloon catheter 10 could be used to deliver a balloon expanded stent, without departing from the present disclosure, especially delivery of a stent to a curved passageway. In addition, the balloon catheter 10 could be utilized in any application known in the art where balloon catheters are utilized, such as for angioplasty, urinary, digestive, reproductive, otolaryngological procedures, etc.

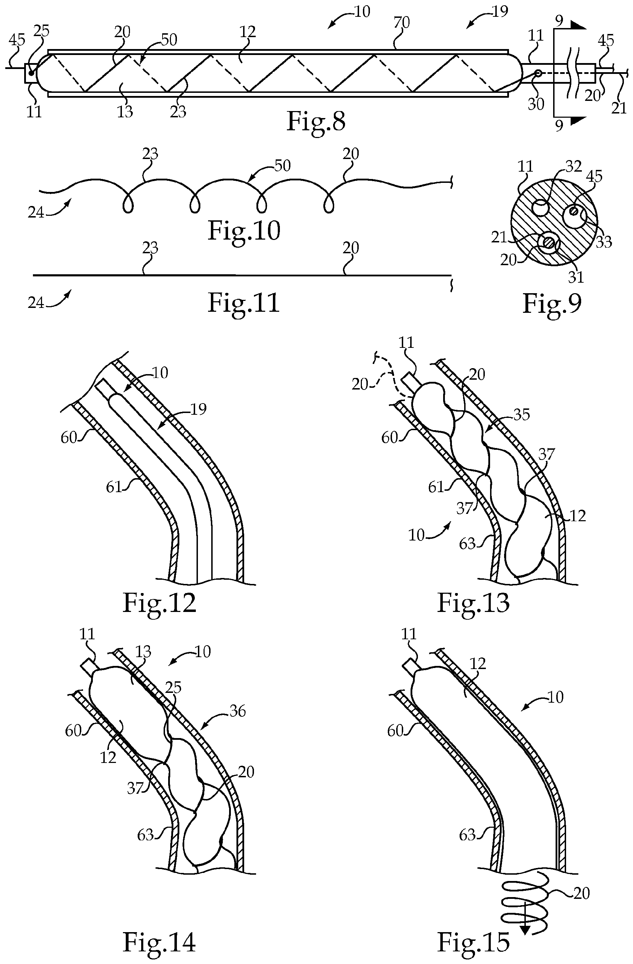

Referring now to Figs. 8 and 9, a shape controlled balloon catheter 10 is shown with the same named features shown with the same numerals used earlier. This embodiment differs from the earlier embodiment in that only a single wire 20 is spirally wound about balloon 12. Unlike the earlier embodiment, the distal end 25 of wire 20 is attached to the catheter 11 distally of the balloon 12. Balloon catheter 10 is shown in a deflated state 19. However, by the user adjusting tension in wire 20 by movement of manipulation segment 21 can cause wire 20 to move on the outer surface 13 of the balloon 12. Thus, the radius of the spiral of wire 20 can be manipulated along a continuum to create a continuum of different inflated shapes for balloon 12. The position of, and tension in, wire 20 in general, and manipulation segment 21 in particular are changed to change the shape of the inflated balloon 12. This embodiment also differs in that catheter 11 is shown as a three lumen catheter that includes a separate lumen 31 for manipulation segment 21 of wire 20, an inflation lumen 32 in fluid communication with balloon 12, and a wire guide lumen 33 that slidably receives a wire guide 45 in a conventional manner (Fig. 9). Although the distal segment 23 of wire 20 is spirally wound about balloon 12, the wire 20 may enter lumen 31 through a side port 30. Alternatively, the manipulation segment 21 could remain outside of balloon 12 as in the previous embodiment. The embodiment of Fig. 8 is also of interest for showing a balloon expanded stent 70 mounted on the outer surface of balloon 12 to illustrate one possible usage of shape controlled balloon catheters 10 according to the present disclosure to deliver stents, especially to curved passageways where the wire constrained shape of the balloon allows it to more easily conform to a curved passageway.

Referring now to Figs. 10 and 11, a wire 20 according to the present disclosure may have a shape 24 that changes responsive to a temperature increase above a predetermined temperature. For instance, the wire 20 may first have a pre-set spiral shape as shown in Fig. 10 and then later assume a straight shape as shown in Fig. 11 after achieving an elevated temperature, such as by being electrically heated. Alternatively, the pre-determined temperature may relate to body temperature without departing from the present disclosure. It is important to note that Figs. 10 and 11 show the distal segment 23 of the wire which would be wrapped around a balloon (not shown) when used as part of a shape controlled balloon catheter according to the present disclosure.

As an example, wire 20 could be a nitinol wire that has been heat set in a particular shape 24 (straight or spiral) at a specified (Austenitic finish, Af) temperature, which may be above body temperature. The wire might be cooled below the heat set temperature and deformed to a different shape. Then by using an electrical current, the wire may be heated to change the shape back to its heat set shape. This strategy permits the wire (and device) to have one shape when inserted into the body (or at any point prior to applying heat), and assume another shape upon applying an external heat source. With an Af just above body temperature and potentially with insulation on the nitinol wire, this can be accomplished without heating the wire such that damage to the adjacent cells occurs. Again, this could be used to restrict certain portions of the balloon and possibly only at certain temperatures. Furthermore, the spiral wire 20 might also be heated, possibly by an electric current after a procedure is performed, to straighten the wire and allow it to be pulled more easily out of the catheter or sheath. Once completely inside the catheter or sheath, the electric current could be turned off. Although nitinol is contemplated as one material for wire 20, other materials could also be utilized, including but not limited to other metallic alloys, and in some instances a suture could be considered a wire 20 in accord with the present disclosure, such as in relation to the embodiment of Figs. 8 and 9. Thus, a wire may not necessarily be metallic to fall within the scope of the present disclosure.

Referring to Figs. 12-15, still another embodiment of a shape controlled balloon catheter 10 is illustrated being maneuvered in a deflated state 19 to a site 61 in a curved passageway 60. This embodiment is similar to the embodiment of Figs. 8 and 9 in that it includes only a single wire 20, but is similar to the embodiment of Figs. 1-7 in that the wire 20 is not attached to either the balloon 12 or the catheter 11. After the shape controlled balloon catheter 10 arrives at site 61, the balloon 12 is inflated as shown in Fig. 13. When this is done, the wire 20 constrains the balloon 12 so that the balloon assumes a first shape 35, which allows the balloon to bend about creases 37 defined by wire 20 to easily conform to the curvature 63 of passageway 60. Those skill in the art will appreciate that passageway 60 could be a body passageway such as an artery, or can be an artificial passageway for purposes of demonstration or teaching without departing from the present disclosure. After being inflated to the first shape 35, the wire 20 may be moved along a centerline of the catheter 11 away from balloon 12 so that the inflated balloon assumes a second shape 36 as shown in Fig. 14. In particular, Fig. 14 shows the distal segment of the balloon 12 in an unconstrained inflated shape, whereas a more proximal segment of the balloon still has wire 20 spirally wrapped around balloon 12. Those skilled in the art will appreciate that, because wire 20 can be moved along a continuum of different positions on the outer surface 13 of balloon 12, the balloon 12 may assume a continuum of different inflated shapes, including the unconstrained shape shown in Fig. 13 where the wire 20 has been completely withdrawn out of contact with balloon 12. Those skilled in the art will appreciate that the wire 20 may be moved proximately away from the balloon, but the present disclosure also contemplates scenarios in which the wire is advanced toward a distal end of catheter 11. By changing the position of wire 20 on the outer surface 13 of balloon 12, the flexibility characteristics of the balloon can be altered invivo.

Although wire 20 is shown as being spiral, it is possible that the wire could be flexible and in a straight configuration. It is possible that the wire could be nitinol that is heat set to a straight configuration, and would want to return to that straight configuration, thereby constraining the balloon when pressurized, with the wire is deformed to a spiral shape. In the straight configuration, the wire could be retracted or advanced over the balloon 12, but likely most easily when the balloon is deflated. It is also possible that the wire 20 could be nitinol that is heat set to a spiral of a specified diameter and pitch configuration to constrain the balloon when pressurized. In the spiral configuration the wire 20 could be retracted or advanced over the balloon, but likely most easily when the balloon is partially inflated or deflated. When the balloon is in deflated state, the wire(s) could be holding close to the balloon, but not so tight where friction would make it difficult to advance or retract the wire relative to the balloon's outer surface 13. Thus, the positioning of the wire(s) over the balloon 12 would most likely be done with the balloon deflated. It is also possible that the wire(s) could be positioned where it extends beyond a distal end of the balloon as shown for instance, with the dotted line in Fig. 13.

### Industrial Applicability

The present disclosure finds generally applicability wherever balloon catheters are utilized. The present disclosure finds specific applicability for use in better conforming the inflated balloon to curvature in a passageway by being able to bend about creases 37 created by a constraining wire 20 spirally wrapped about the balloon 12. The present disclosure also finds specific applicability in the ability to change the shape of the balloon invivo to both initially conform to a curved passageway with a wire constraining the shape of the balloon, and then more fully inflating the balloon by moving the wire to unconstrain portions of the balloon, such as for better expanding a stent in place in a curved passageway without tending to straighten the passageway as in the prior art.

The disclosed method may be performed outside the body, for example for training or demonstration purposes, utilising a model providing a passageway representing a passageway in a patient's body.

It should be understood that the above description is intended for illustrative purposes only, and is not intended to limit the scope of the present disclosure in any way. Thus, those skilled in the art will appreciate that other aspects of the disclosure can be obtained from a study of the drawings, the disclosure and the appended claims.

## Claims

1. A shape controlled balloon catheter comprising
a catheter;
a balloon mounted about the catheter;
a wire spirally wound about the balloon and including a manipulation segment extending along a centerline of the catheter away from the balloon;
wherein a position of the wire on an outer surface of the balloon changes responsive to a movement of the manipulation segment relative to the catheter along the centerline;
the balloon having a continuum of different inflated shapes corresponding to a continuum of different positions of the manipulation segment relative to the catheter along the centerline.

2. The shape controlled balloon catheter of claim 1 wherein a distal end of the wire is attached to the catheter distally of the balloon.

3. The shape controlled balloon catheter of claim 1 wherein a distal end of the wire is moved along an outer surface of the balloon responsive to the movement of the manipulation segment relative to the catheter along the centerline.

4. The shape controlled balloon catheter of any previous claim wherein the wire is a first wire; and
a second wire spirally wound about the balloon and including a manipulation segment extending along the centerline away from the balloon.

5. The shape controlled balloon catheter of claim 4 wherein the second wire is wound in an opposite direction about the balloon than the first wire.

6. The shape controlled balloon catheter of any previous claim wherein the manipulation segment enters through a side port of the catheter and extends inside of a lumen of the catheter.

7. The shape controlled balloon catheter of any previous claim including the catheter slidably received in a sheath; and
the manipulation segment extends along the centerline between an outer surface of the catheter and an inner surface of the sheath.

8. The shape controlled balloon catheter of any previous claim wherein a distal segment of the wire has a preset spiral shape with a diameter that is smaller than an inflation diameter of the balloon.

9. The shape controlled balloon catheter of any previous claim wherein a distal segment of the wire has a shape that changes responsive to a temperature increase above a predetermined temperature.

10. A method of operating a shape controlled balloon catheter comprising the steps of:
positioning a balloon of the shape controlled balloon catheter at a site in a passageway while in a deflated state;
inflating the balloon;
constraining the balloon during the inflation step with a wire spirally wound about the balloon so that the inflated balloon has a first shape;
changing the inflated balloon from the first shape to a second shape responsive to moving a manipulation segment of the wire extending along a centerline of the catheter away from the balloon.

11. The method of claim 10 including conforming the balloon to a curvature of a passageway by bending the balloon about crease in the inflated balloon defined by the wire.

12. The method of any of claims 10-11 wherein the step of moving the manipulation segment includes moving a distal end of the wire along an outer surface of the balloon; and
the second shape includes a distal segment of the balloon out of contact with the wire.

13. The method of any of claims 10-12 wherein the wire is a first wire;
a second wire spirally wound about the balloon and including a manipulation segment extending along the centerline away from the balloon; and
moving a manipulation segment of the second wire, preferably wherein the second wire is wound in an opposite direction about the balloon than the first wire.

14. The method of any of claims 10-13 wherein the step of moving the manipulation segment includes moving the manipulation segment through a side port of the catheter.

15. The method of any of claims 10-14 including the catheter being slidably received in a sheath; and
the step of moving the manipulation segment includes moving the manipulation segment along the centerline between an outer surface of the catheter and an inner surface of the sheath.
